# EUROPEAN PATENT APPLICATION

(11) **EP 4 083 138 A1**
(43) Date of publication of application: **02.11.2022**
(21) Application number: 20906582.0
(22) Date of filing: 18.12.2020
(51) Int. Cl.: C08L 83/04, C08K 3/36, D06M 15/643

(54) **FIBER TREATMENT AGENT COMPOSED OF AQUEOUS DISPERSION OF AQUEOUS SILICONE ELASTOMER, AND FILM**

(30) Priority: 27.12.2019 JP 2019238803
(71) Applicant: Shin-Etsu Chemical Co., Ltd., Tokyo 100-0005 (JP)
(72) Inventor: TAWATA, Hanako, Annaka-shi, Gunma 379-0224 (JP)
(74) Representative: V.O.
(86) International application number: PCT/JP2020/047416
(87) International publication number: WO 2021/132073

(57) **Abstract**

One of the objects of the present invention is to provide a fiber treatment agent consisting of an aqueous silicone dispersion capable of quickly forming a silicone elastomer coating having elongation and strength on drying at room-temperature, and to provide a coating which is the dry product. Further, the object of the present invention is to provide a coating on drying at room-temperature without using a high activity catalyst such as organotin compounds. The present invention provides a fiber treatment agent comprising 100 parts by mass of (A) a silicone elastomer which is an addition-reaction product of the following component (A-1) with the following components (A-2) and (A-3):
(A-1) an alkenyl group-containing organopolysiloxane having two or more alkenyl groups per molecule,
(A-2) an organohydrogen polysiloxane having three or more hydrosilyl groups per molecule,
(A-3) a linear diorganohydrogen polysiloxane having hydrosilyl groups only at both molecular chain terminals;
0.1 to 8 parts by mass of at least one surfactant selected from (B) anionic surfactants and (C) nonionic surfactants,
3 to 35 parts by mass of (D) colloidal silica, and
15 to 200 parts by mass of (E) water, relative to total 100 parts by mass of components (A) and (D).

## Description

### TECHNICAL FIELD

The present invention relates to a fiber treatment agent comprising an aqueous dispersion of a silicone elastomer which easily forms a coating by drying at room-temperature, and a coating obtained by drying the fiber treatment agent.

### BACKGROUND ART

Conventional aqueous silicone dispersions which form elastomer coatings upon drying have various compositions. These dispersions have been used as fiber treatment agents, rubber coating agents, building member coating agents, coating agents of paper or plastic coating, or additives thereof for the purpose of imparting lubricity, water repellency or releasability.

As an example of a composition which forms an elastomer coating upon drying, Japanese Application Laid-Open No. Sho 53-130752 (Patent Literature 1) describes an emulsion composition comprising a polydiorganosiloxane capped with hydroxyl groups at both ends of the molecular chain, a silane having at least three hydrolyzable groups, and a curing catalyst. However, the composition requires heating at 100 to 300°C to form an elastomer coating by simultaneously drying and subjecting silicone to a condensation reaction and, therefore, on drying at room-temperature, the reaction rate is slow or no reaction takes place. Treatment at high temperatures may damage the fibers and, therefore, it is preferable to be able to perform treatment at room temperature.

Japanese Application Laid-Open No. Sho 54-131661 (Patent Literature 2) discloses an organopolysiloxane latex composition obtained by the emulsion polymerization of a cyclic organosiloxane and an organotrialkoxysilane. However, according to the method, the manufacturing cost is high because a long polymerization process is required. Recently, there has been a demand for products in which the amount of octamethylcyclotetrasiloxane, decamethylcyclopentasiloxane, and dodecamethylcyclohexanesiloxane is suppressed, whereas there is the problem that the aforementioned composition comprises a large amount of cyclic siloxane oligomer.

In addition to the above, as an example of the composition which forms an elastomer coating on drying, Japanese Application Laid-Open No. Hei 8-85760 (Patent Literature 3) discloses an emulsion composition comprising an alkoxy group or a hydroxyl group-containing chain organopolysiloxane, an Si-H bond-containing organopolysiloxane, silica or polysilsesquioxane, an amide group or a carboxyl group-containing organoalkoxysilane, an epoxy group or an amino group-containing organoalkoxysilane, and a curing catalyst.

Japanese Application Laid-Open No. Hei 11-158380 (Patent Literature 4) discloses an emulsion composition comprising a hydroxyl group or an alkoxy group end-capped branched organopolysiloxane, an organopolysiloxane having two or three hydroxyl or alkoxy groups bonded to a silicon atom, powdered silica and a curing promoter.

Japanese National Phase Publication No. Sho 56-501488 (Patent Literature 5) discloses an emulsion composition comprising a hydroxyl endblocked polydiorganosiloxane containing vinyl-substituted siloxane units, which was treated such that crosslinking takes place by forming radicals within the siloxane.

Japanese Application Laid-Open No. Hei 7-196984 (Patent Literature 6) discloses a silicone emulsion composition obtained by mixing and dispersing an emulsion of an amino group-containing organopolysiloxane and an epoxy-containing hydrolyzable silane, or a silicone emulsion composition obtained by mixing and dispersing an emulsion of an epoxy group-containing organopolysiloxane and an amino group-containing hydrolyzable silane. These silicone emulsion compositions are cured through the removal of water. However, in order to satisfactorily form a cured rubber coating within a short time, organotin compounds having high catalytic activity must be used. However, the use of tin compounds is recently being avoided due to their toxicity.

As an example of a composition which forms a silicone elastomer coating by an addition-reaction of alkenylsilyl groups with hydrosilyl groups while drying, Japanese Application Laid-Open No. Sho 50-94082 (Patent Literature 7) discloses an emulsion comprising a polydiorganosiloxane capped with vinyl group at an end of the molecular chain, a polyorganohydrogen polysiloxane and a platinum catalyst.

Japanese Application Laid-Open No. Sho 54-52160 (Patent Literature 8) discloses an emulsion comprising a polydiorganosiloxane containing vinyl group at an end or side chain of the molecular chain, a polysiloxane having silicon-bonded hydrogen, colloidal silica and a platinum catalyst. However, these compositions have the problem that a reaction will take place or hydrogen gas will be generated with the lapse of time if the hydrosilyl group containing siloxane and the platinum catalyst coexist. Therefore, the silicone emulsion and the platinum catalyst must be mixed just prior to use. However, for general consumers using a fiber treatment agent, the useability deteriorates when they use the composition after mixing the liquids.

Japanese Application Laid-Open No. Sho 56-36546 (Patent Literature 9) discloses a method of adding a platinum catalyst to an emulsion comprising a polydiorganosiloxane capped with vinyl groups at both ends of the molecular chain and a polyorganohydrogensiloxane to, thereby, prepare an emulsion composition comprising a crosslinked silicone elastomer by the addition-reaction of vinylsilyl groups with hydrosilyl groups, and a method of further blending colloidal silica in the emulsion composition. The addition-reaction of vinylsilyl groups with hydrosilyl groups in the emulsion composition disclosed in Patent Literature 9 is completed and forms an elastomer coating on drying. However, there are the drawbacks that the resulting coating is not extensible and is brittle.

### PRIOR LITERATURE

### Patent Literature

[Patent Literature 1] Japanese Application Laid-Open No. Sho 53-130752(1978)
[Patent Literature 2] Japanese Application Laid-Open No. Sho 54-131661(1979)
[Patent Literature 3] Japanese Application Laid-Open No. Hei 8-85760(1996)
[Patent Literature 4] Japanese Application Laid-Open No. Hei 11-158380(1999)
[Patent Literature 5] Japanese National Phase Publication No. Sho 56-501488(1981)
[Patent Literature 6] Japanese Application Laid-Open No. Hei 7-196984(1995)
[Patent Literature 7] Japanese Application Laid-Open No. Sho 50-94082(1975)
[Patent Literature 8] Japanese Application Laid-Open No. Sho 54-52160(1979)
[Patent Literature 9] Japanese Application Laid-Open No. Sho 56-36546(1981)

### SUMMARY OF THE INVENTION

### PROBLEMS TO BE SOLVED BY THE INVENTION

In consideration of the aforementioned circumstances, one of the objects of the present invention is to provide a fiber treatment agent consisting of an aqueous silicone dispersion capable of quickly forming a silicone elastomer coating having elongation and strength by drying at room-temperature, and to provide a coating which is the dry product. Further, the object of the present invention is to provide a coating by drying at room-temperature without using a high activity catalyst such as organotin compounds.

### MEANS TO SOLVE THE PROBLEMS

The present inventors have conducted research and found that the objects are attained by the following:
a fiber treatment agent which is an aqueous dispersion of a silicone elastomer capable of forming a coating necessarily includes an organohydrogen polysiloxane having three or more hydrosilyl groups per molecule, and a linear diorganohydrogen polysiloxane having hydrosilyl groups only at both molecular chain terminals, as the raw materials of a silicone elastomer which is an addition-reaction product,
the amount of anionic surfactant and/or nonionic surfactant which is an emulsifier contained in the aqueous dispersion is minimized, and
the fiber treatment agent comprises a specific amount of colloidal silica.

That is, the present invention provides a fiber treatment agent comprising 100 parts by mass of (A) a silicone elastomer which is an addition-reaction product of the following component (A-1) with the following components (A-2) and (A-3):
(A-1) an alkenyl group-containing organopolysiloxane having two or more alkenyl groups per molecule,
(A-2) an organohydrogen polysiloxane having three or more hydrosilyl groups per molecule,
(A-3) a linear diorganohydrogen polysiloxane having hydrosilyl groups only at both molecular chain terminals;
   0.1 to 8 parts by mass of at least one surfactant selected from (B) anionic surfactants and (C) nonionic surfactants,
   3 to 35 parts by mass of (D) colloidal silica, and
   15 to 200 parts by mass of (E) water, relative to total 100 parts by mass of components (A) and (D).

The present invention further provides a coating which is a dry product of the fiber treatment agent.

### SUMMARY OF THE INVENTION

The fiber treatment agent of the present application quickly forms a silicone elastomer coating having a suitable elongation and strength on drying at room-temperature. Therefore, a good fiber treatment coating can be provided on drying at room-temperature without using a high activity catalyst such as an organotin compound.

### DETAILED DESCRIPTION OF PREFERRED EMBODIMENTS

The fiber treatment agent of the present application is an aqueous silicone aqueous dispersion in which a silicone elastomer is dispersed in water. Note that, in the present invention, the term "aqueous" means that a dispersion is readily diluted with water.

The present invention will be described in detail below.

### [(A) Silicone Elastomer]

The (A) silicone elastomer is an addition reaction product of an alkenyl group-containing organopolysiloxane with an organopolysiloxane having a hydrosilyl group. The alkenyl group-containing organopolysiloxane is an alkenyl group-containing organopolysiloxane (A-1) having two or more alkenyl groups per molecule. The organopolysiloxane having a hydrosilyl group is an organopolysiloxane (A-2) having at least three hydrosilyl groups per molecule and a linear diorganopolysiloxane (A-3) having hydrosilyl groups at both ends of the molecular chain. In component (A), the hydrosilyl groups of the components (A-2) and (A-3) and the alkenyl group of the component (A-1) are bound by an addition-reaction. In order for the fiber treatment agent thus obtained to be formed into a silicone elastomer coating, component (A) must be made into a lattice shape. However, with only (A-1) organopolysiloxane having two alkenyl groups and (A-3) linear diorganopolysiloxane having hydrosilyl groups at both ends of the molecular chain, the resulting siloxane only has a linear structure, and does not form a coating. If only a coating is formed, the fiber treatment agent may comprise the (A-1) aforementioned components and only (A-2) organopolysiloxane having at least three hydrosilyl groups. However, by inserting a structure derived from the aforementioned component (A-3), the lattice density of the coating can be adjusted. It is preferable to reduce the lattice density to some extent in order to provide a coating having a suitable elongation.

Components (A-1) to (A-3) will be described below.

### Component (A-1)

Conventionally known alkenyl group-containing organopolysiloxanes having two or more alkenyl groups per molecule may be used alone or in appropriate combinations of two or more. For example, component (A-1) may be represented by the following average compositional formula (1):

R¹ₐR²_{b}SiO_{(4-A-b)/2} (1)

wherein R¹ is, independently of each other, a substituted or unsubstituted monovalent hydrocarbon group having 1 to 30 carbon atoms, exclusive of alkenyl group; and R² is, independently of each other, an alkenyl group having 2 to 6 carbon atoms. a and b are positive numbers satisfying equations 0 < a < 3, 0 < b ≤ 3, and 0.1 ≤ a + b ≤ 3, and are preferably positive numbers satisfying equations 0 < a ≤ 2.295, 0.005 ≤ b ≤ 2.3, and 0.5 ≤ a + b ≤ 2.3. The viscosity of the organopolysiloxane is not limited to a specific one, and the organopolysiloxane may contain a solid-state compound. The kinematic viscosity of the organosiloxane at 25°C is preferably 100,000 mm²/s or less, more preferably 10,000 mm²/s or less. The lower limit is not particularly limited, but is 10 mm²/s or more, and preferably 50 mm²/s or more.

Examples of R¹ include alkyl groups such as methyl, ethyl, propyl, butyl, pentyl, hexyl, heptyl, octyl, decyl, undecyl, dodecyl, tetradecyl, pentadecyl, hexadecyl, heptadecyl, octadecyl, nonadecyl, icosyl, heneicosyl, docosyl, tricosyl, tetracyl, and triacontyl; aryl groups such as phenyl, tolyl, and naphthyl; aralkyl groups such as benzyl and phenethyl; cycloalkyl groups such as cyclopentyl, cyclohexyl and cycloheptyl; and those hydrocarbon groups wherein a part or all of the hydrogen atoms bonded to a carbon atom of these groups is substituted with halogen atoms (such as fluorine, chlorine, bromine and iodine atoms) and/or a substituent such as acryloyloxy, methacryloyloxy, epoxy, glycidoxy or carboxyl group. Among them, in view of industrial considerations, it is preferable that at least 50 mol% of the R¹ groups is a methyl group.

Examples of R² include vinyl, allyl, propenyl, butenyl, pentenyl, and hexenyl groups, and in view of industrially, a vinyl group is preferable.

The structure of the component (A-1) may be linear, cyclic or branched. An organopolysiloxane having a linear structure is, for example, represented by the following general formula (2): 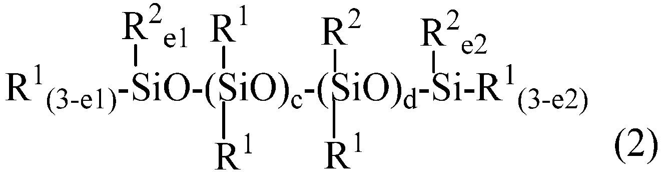 wherein R¹ and R² are as defined above; c is a positive number; d is 0 or a positive number; e1 and e2 are, independently of each other, 0 or 1; and the upper limit of c and d is not limited, but the siloxane represented by general formula (2) has a kinematic viscosity at 25°C of 100,000 mm²/s or less, preferably 10,000 mm²/s or less. d, e1, and e2 are values which satisfy equation d + e1 + e2 ≥ 2.

An organopolysiloxane having a branched structure is, for example, represented by the following general formula (3) having branches from an R¹ SiO_{3/2} unit: 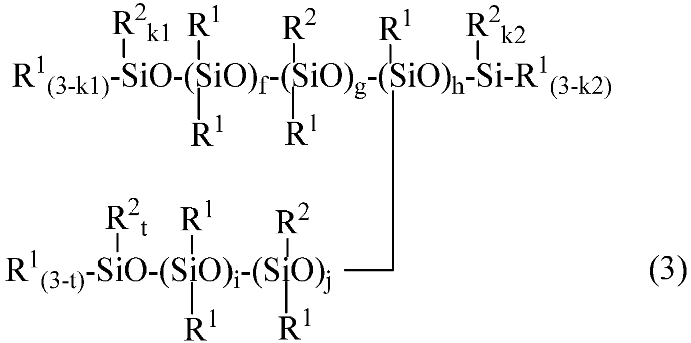 wherein R¹ and R² are as defined above; f is a positive number; g is 0 or a positive number; h is a positive number; i is a positive number; j is 0 or a positive number; and k1 and k2 are, independently of each other, 0 or 1; t is 0 or 1, with the proviso that g, j, and t are values such that the kinematic viscosity of the siloxane at 25°C is 100,000 mm²/s or less, preferably 10,000 mm²/s or less, and g, j, h, k1, k2 and t are values which satisfy equation g + h × (j + t) + k1 + k2 ≥ 2.

An example of the organopolysiloxane having branches from an SiO_{4/2} unit includes the compound represented by the following general formula (4): 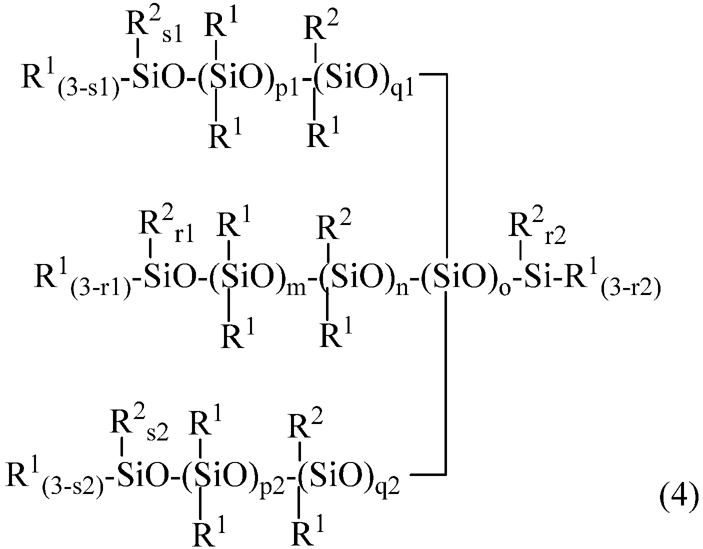 wherein R¹ and R² are as defined above; m is a positive number; n is 0 or a positive number; o is a positive number; p1 is a positive number; p2 is a positive number; q1 is 0 or a positive number; and q2 is 0 or a positive number, with the proviso that, m, n, o, p1, p2, and q1 are values such that the kinematic viscosity of the siloxane at 25°C is 100,000 mm²/s or less and preferably 10,000 mm²/s or less, r1 and r2 are, independently of each other, 0 or 1, and s1 and s2 are, independently of each other, 0 or 1, however, are values which satisfy equation n + "o" x (q1 + q2 + s1 + s2) + r1 + r2 ≥ 2.

The organopolysiloxane having two or more alkenyl groups per molecule may be represented by the following unit formula (5);

[R¹₃ SiO_{1 / 2} ]ₜ [R²(R¹)₂ SiO_{1 / 2} ]ᵤ [SiO₄ / ₂ ]ᵥ (5)

wherein R¹ and R² are as defined above; t is 0 or a positive number of 300 or less; u is a positive number of 300 or less; and v is a positive number of 300 or less.

### Component (A-2)

Conventionally known organopolysiloxanes having at least 3 hydrosilyl groups per molecule may be used alone or in appropriate combinations of two or more, and for example, may be represented by the following average compositional formula (6):

R³_{w}HₓSiO_{(4-w-x)/2} (6)

wherein R³ is, independently of each other, a substituted or unsubstituted monovalent hydrocarbon group having 1 to 30 carbon atoms, exclusive of alkenyl group. w and x are a positive number which satisfies equation 0 < w < 3, 0 < x ≤ 3, and 0.1 ≤ w + x ≤ 3, preferably a positive number which satisfies equation 0 < w ≤ 2.295, 0.005 ≤ x ≤ 2.3, and 0.5 ≤ w + x ≤ 2.3. The viscosity of the organohydrogen polysiloxane is not particularly limited, and the organopolysiloxane may contain a solid-state compound. The kinematic viscosity of the organohydrogen polysiloxane at 25°C is preferably 100,000 mm²/s or less, and more preferably 10,000 mm²/s or less. The lower limit is not particularly limited, and is 10 mm²/s or more, preferably 50 mm²/s or more. Examples of R³ include the same groups as those described for R¹ above. R³ is preferably a methyl group.

The structure of component (A-2) may be linear, cyclic or branched. The organohydrogen polysiloxane having a linear structure is, for example, represented by the following general formula (7): 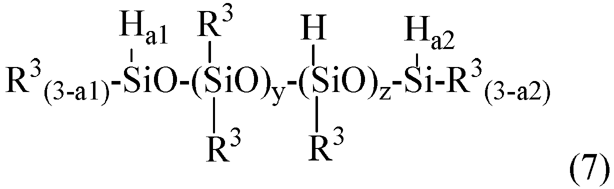 wherein R³ are as defined above; y is a positive number; and z is a positive number, with the proviso that, the kinematic viscosity of the siloxane at 25°C is 100,000 mm²/s or less, preferably 10,000 mm²/s or less, and a1 and a2 are, independently of each other, 0 or 1, however, z and a1 are the numbers which satisfy equation z + a1 + a2 ≥ 3.

The organohydrogen polysiloxane having a branched structure from an R³ SiO_{3/2} unit is, for example, represented by the following general formula: 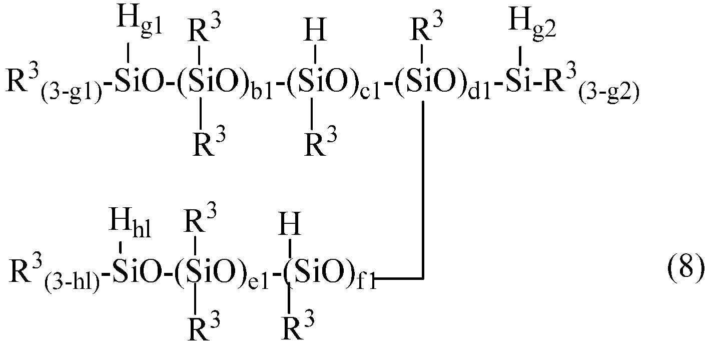 wherein R³ is as defined above; b1 is a positive number; c1 is 0 or a positive number; d1 is a positive number; e1 is a positive number; f1 is 0 or a positive number; and b1, c1, d1, e1, and f1 are the numbers such that the kinematic viscosity at 25°C is 100,000 mm²/s or less, preferably 10,000 mm²/s or less; g1 and g2 are, independently of each other, 0 or 1; and h1 is 0 or 1; and c1, d1, f1, g1, g2 and h1 are the numbers which satisfy equation c1 + d1 × (f1 + h1)+ g1 + g2 ≥ 3.

Examples of structures which branch from a SiO_{4/2} unit include the compound represented by the following general formula: 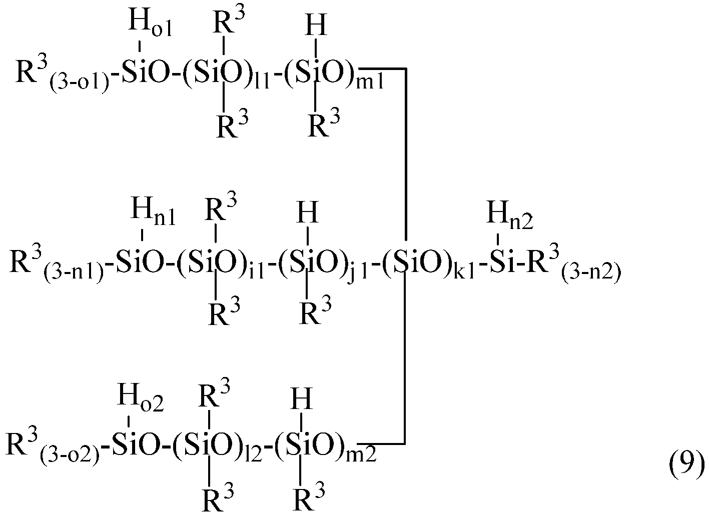 wherein R³ is as defined above; i1 is a positive number; j1 is 0 or a positive number; k1 is a positive number; 11 is a positive number; m1 is 0 or a positive number; m2 is 0 or a positive number, with the proviso that the kinematic viscosity of the siloxane at 25°C is 100,000 mm²/s or less and preferably 10,000 mm²/s or less; n1 is 0 or 1; o1 is 0 or 1; and j1, k1, m1, m2, n1, n2, o1 and o2 are the numbers which satisfy equation j1 + k1 × (m1 + m2 + o1 + o2) + n1 + n2 ≥ 3.

Structures having at least three hydrosilyl groups per molecule represented by the following unit formula (10) may also be included:

[R³₃ SiO_{1 / 2}]_{p 1} [H(R³)₂ SiO_{1/ 2}]_{q 1} [SiO_{4 / 2}]_{r 1} (10)

wherein R³ is as defined above; p1 is 0 or a positive number of 300 or less; q1 is a positive number of 300 or less; and r1 is a positive number of 300 or less.

### Component (A-3)

Conventionally known linear diorganopolysiloxane having hydrosilyl groups at both ends of the molecular chain may be used alone or in appropriate combinations of two or more. For example, the diorganohydrogen polysiloxane is represented by the following general formula (11): 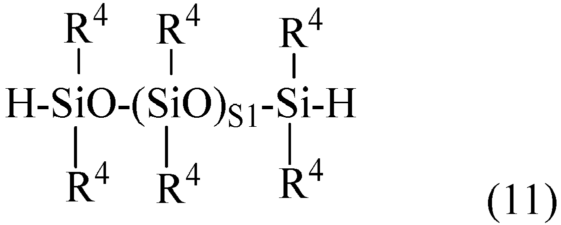 wherein R⁴ is, independently of each other, a substituted or unsubstituted monovalent hydrocarbon groups having 1 to 30 carbon atoms, exclusive of alkenyl group; s1 is a positive number from 5 to 1,000, and preferably is a positive number from 10 to 500.

Examples of R⁴ include the same groups as those described for R¹ above. R⁴ is preferably a methyl group.

The aforementioned component (A-3) preferably has a kinematic viscosity at 25°C of 10,000 mm²/s or less, more preferably 5,000 mm²/s or less, and even more preferably 1,000 mm²/s or less. The lower limit is not particularly limited, but may be 10 mm²/s or more, and is preferably 40 mm²/s or more.

The mixed and dissolved solution of the aforementioned component (A-1), component (A-2), and component (A-3) preferably has a kinematic viscosity at 25°C of 10,000 mm²/s or less, more preferably 1,000 mm²/s or less. If the kinematic viscosity of the mixed solution exceeds 10,000 mm²/s, it may become difficult to reduce the particle size in the preparation method to be described later. Note that, in the present invention, the kinematic viscosity is determined at 25°C by an Ostwald viscometer.

The blending ratio of the aforementioned component (A-1), component (A-2) and component (A-3) is preferably the weight ratio such that the ratio of the total number of hydrosilyl groups in components (A-2) and (A-3) relative to the number of alkenyl groups in component (A-1) is 0.5 to 2.0, more preferably in the range from 0.8 to 1.5.

The mass ratio of the aforementioned component (A-2) and component (A-3) is preferably in the range from (A-2):(A-3) = 5:95 to 90:10 (mass ratio), more preferably from 10:90 to 80:20 (mass ratio). If the mass ratio of component (A-3) relative to the mass of component (A-2) is too low, the coating thus obtained may not be able to elongate and may be brittle. If the ratio of component (A-3) is too high, the coating is not formed and a gel or liquid may form.

Components (A-1), (A-2) and (A-3) may contain octamethylcyclotetrasiloxane, decamethylcyclopentasiloxane, and dodecamethylcyclohexanesiloxane as impurities, but the content may be no more than 1000 mass ppm, preferably no more than 500 mass ppm, respectively, relative to the total mass of components (A-1), (A-2) and (A-3). The lower limit is not particularly limited, a small amount is the better and the content may be 0 mass ppm.

### [Platinum group metal catalyst]

As stated above, the (A) silicone elastomer in the present invention is the addition-reaction product of the alkenyl group-containing organopolysiloxane [component (A-1)] and the organopolysiloxane having a hydrosilyl group [components (A-2) and (A-3)]. A platinum group metal catalyst may be used in the addition reaction. Conventionally known platinum metal base catalysts as the addition-reaction catalyst may be used. Examples of catalysts include elemental platinum group metals such as platinum (inclusive of platinum black), rhodium and palladium; platinum chloride, chloroplatinic acid and chloroplatinic acid salts such as H₂ PtCl4 • kH₂ O, H₂ PtCl₆ • kH₂ O, NaHPtCl₆ • kH₂ O, KHPtCl₆ • kH₂ O, Na₂ PtCl₆ • kH₂ O, K₂ PtCl4 • kH₂ O, PtCl4 • kH₂ O, PtCl₂, and Na₂ HPtCl₄ • kH₂ O (with the proviso that, wherein, k is an integer from 0 to 6, and is preferably 0 or 6); alcohol-modified chloroplatinic acid (see U.S. Pat. No. 3,220,972); complexes of platinum chloride or chloroplatinic acid with olefins (see U.S. Pat. Nos. 3,159,601; 3,159,662; and 3,775,452), complexes of chloroplatinic acid with vinyl-containing siloxanes, and complexes of platinum with vinyl-containing siloxanes; platinum group metals such as platinum black and palladium on carriers such as alumina, silica and carbon; olefin complexes with rhodium and chlorotris(triphenylphosphine)rhodium known as Wilkinson catalyst.

The amount of the platinum group metal catalyst may be an effective amount (a so-called catalytic amount) for promoting the addition reaction. For example, the amount of the platinum-containing catalyst may be such that the mass amount of platinum in the catalyst is in the range from 0.1 to 100 ppm (mass), preferably in the range from 0.5 to 50 ppm, even more preferably in the range from 1 to 30 ppm, relative to the total weight of component (A-1), component (A-2) and component (A-3).

The silicone elastomer (A) may contain a silicone oil, a silicone resin, an organosilane, an inorganic powder, an organic powder, and an antioxidant, in addition to the aforementioned addition-reaction products.

As stated above, the silicone elastomer (A) is the addition-reaction product of component (A-1) with components (A-2) and (A-3), and the structure of the silicone elastomer thus obtained may be appropriately determined depending on the type and the blending ratio of components (A-1), (A-2) and (A-3).

The aforementioned silicone elastomer (A) may be obtained by subjecting components (A-1), (A-2) and component (A-3) to an addition reaction in the presence of a surfactant and water which will be described below. Therefore, the silicone elastomer thus obtained is in a particle form dispersed in water. The volume average particle size is preferably no more than 10 µm, and more preferably no more than 1 µm. The lower limit is not particularly limited, but may be set to 0.1 µm or more. If the volume average particle size is larger than 10 µm, the coating to be formed cannot be elongated and becomes brittle. Note that, in the present invention, volume average particle size is determined by the laser diffraction/scattering type particle size measuring method.

The silicone aqueous dispersion of the present invention contains at least one surfactant selected from (B) anionic surfactants and (C) nonionic surfactants as a dispersant of the aforementioned (A) silicone elastomer. The amount of the surfactant is 0.1 to 8 parts by mass, preferably 0.5 to 7 parts by mass, more preferably 1.0 to 6 parts by mass, relative to 100 parts by mass of the aforementioned component (A). If the amount of the surfactant is within the aforementioned range, component (A-1), component (A-2), and component (A-3) can be emulsified in water in the aforementioned addition reaction, and the particle size of the silicone elastomer thus obtained can be made sufficiently small.

### [(B) Anionic Surfactant]

The (B) anionic surfactant functions as the dispersant of the (A) silicone elastomer in an aqueous dispersion of an aqueous silicone, and also functions as an emulsifier in the emulsification of the aforementioned component (A-1) and components (A-2) and (A-3). The anionic surfactant may be used alone or in an appropriate combination of two or more.

Examples of the anionic surfactant include alkyl sulfate ester salts such as sodium lauryl sulfate, polyoxyethylene alkyl ether sulfate ester salt, polyoxyethylene alkyl phenyl ether sulfate ester salt, sulfate ester salt of fatty acid alkyrrole amide, alkylbenzene sulfonate, polyoxyethylene alkyl phenyl ether sulfonate, α-olefin sulfonate, α-sulfonate fatty acid ester salt, alkylnaphthalene sulfonic acid, alkyl diphenyl ether disulfonate, alkane sulfonate, n-acyltaurate, dialkylsulfosuccinate, monoalkylsulfosuccinate, polyoxyethylene alkyl ether sulfosuccinate, fatty acid salt, polyoxyethylene alkyl ether carboxylic acid salt, n-acylamino acid salt, monoalkyl phosphate ester salt, dialkyl phosphate ester salt, and polyoxyethylene alkyl ether phosphate ester salt. From the viewpoint of the elongation and the tensile strength of the elastomer coating, alkyl sulfate salts are preferable.

The amount of component (B) is 0 to 5 parts by mass, preferably 0 to 2 parts by mass, relative to 100 parts by mass of component (A). If the amount is larger than 5 parts by mass, the coating thus obtained is not extensible and becomes brittle. The lower limit of component (B) may be 0 parts by mass, and the total of component (B) and component (C) may be in the range of the aforementioned total amount. When component (B) is contained, the amount of component (B) is preferably 0.05 parts by mass or more.

### [(C) Nonionic Surfactant]

The (C) nonionic surfactant functions as the dispersant of component (A) silicone elastomer in the aqueous silicone dispersion. Component (C) is also an emulsifier in the emulsification of the alkenyl group-containing organopolysiloxane and the organopolysiloxane having hydrosilyl group as the raw material of component (A) which will be described later in detail and a dispersant of the platinum group metal catalyst.

Examples of the nonionic surfactant include polyoxyethylene alkyl ethers, polyoxyethylene polyoxypropylene alkyl ethers, polyoxyethylene alkyl phenyl ethers, polyethylene glycol fatty acid esters, polyoxyethylene sorbitan fatty acid esters, polyoxyethylene sorbitol fatty acid esters, polyoxyethylene glycerol fatty acid esters, polyglycerol fatty acid esters, propylene glycol fatty acid esters, polyoxyethylene castor oil, polyoxyethylene hydrogenated castor oil, polyoxyethylene hydrogenated castor oil fatty acid esters, polyoxyethylene alkyl amines, polyoxyethylene fatty acid amides, polyoxyethylene-modified organopolysiloxanes, and polyoxyethylene polyoxypropylene-modified organopolysiloxanes. The nonionic surfactant may be used alone or in a combination of two or more. When two or more surfactants are used in combination, a polyether-free nonionic surfactant such as sorbitan fatty acid ester or glycerol fatty acid ester may be used in combination.

The nonionic surfactant acts to reduce the coating-forming ability, so that a smaller amount is preferable. The amount of component (C) is 0 to 8 parts by mass, preferably 0 to 6 parts by mass, and even more preferably 0 to 4 parts by mass, relative to 100 parts by mass of component (A). The lower limit of component (C) may be 0 parts by mass, the total of component (B) and component (C) may be in the range of the aforementioned total amount. The amount of component (C) is preferably 0.05 parts by mass or larger.

Either component (B) or component (C) may be used alone or in combination. When they are used in combination, the ratio may be arbitrarily determined. The higher the ratio of component (B), the better the coating forming, and a coating having an excellent tensile strength and an excellent elongation is obtained. However, if the liquid of the blending system is basic, it may separate and the stability may decrease. Component (C) is nonionic, so that it is stable in a mixture with either anionic or cationic compounds. Therefore, component (C) may be added to a liquid having desired properties.

### [(D) Colloidal Silica]

Component (D) is colloidal silica which functions to improve the elongation and the tensile strength of the silicone elastomer coating. The colloidal silica is a nano-sized silica and has the particle size of preferably from 10 to 300 nm, more preferably from 10 to 50 nm. Note that, the measurement of the particle size is performed by observation with a transmission electron microscope.

In the present invention, the colloidal silica may be used as a water dispersion type of a so-called silica sol (aqueous dispersion of colloidal silica). The concentration of colloidal silica in the aqueous dispersion is not particularly limited, and for example, may be from 10 to 60 mass%. The pH is not particularly limited, but a pH in the range from 4 to 10 is preferable from the viewpoint of safety of use.

The amount of component (D) is from 3 to 35 parts by mass, preferably from 5 to 25 parts by mass, relative to 100 parts by mass of the (A) silicone elastomer. If the amount is less than the aforementioned lower limit, the strength of the coating thus obtained weakens. If the amount is excess of the aforementioned upper limit, the coating thus obtained is not elongated.

### [(E) Water]

The (E) water in the present invention is a dispersing medium for the (A) silicone elastomer and the (D) colloidal silica. The amount of component (E) is from 15 to 200 parts by mass, preferably from 25 to 150 parts by mass, relative to a total of 100 parts by mass of components (A) and (D). If the amount is in excess of 200 parts by mass, the drying rate is slow and it takes more time for coating formation. If the amount of component (E) is less than 15 parts by mass, the viscosity of the silicone aqueous dispersion increases, and the manufacturing and the handling thereof become difficult.

### [Other Components]

The fiber treatment agent of the present invention may comprise a water-soluble polymer compound for the purpose of improving the dispersibility of the (A) silicone elastomer. The water-soluble polymer compound is not particularly limited, and nonionic water-soluble polymer compounds, anionic water-soluble polymer compounds, cationic water-soluble polymer compounds, and zwitterionic water-soluble polymer compounds may be used.

Examples of the nonionic water-soluble polymer compound includes copolymers of vinyl alcohol with vinyl acetate, acrylamide polymers, vinyl pyrrolidone polymers, copolymers of vinyl pyrrolidone with vinyl acetate, polyethylene glycol, isopropylacrylamide polymers, methyl vinyl ether polymers, starch, methyl cellulose, hydroxyethyl cellulose, hydroxypropyl cellulose, guar gum, and xanthane gum.

Examples of the anionic water-soluble polymer compound include sodium acrylate polymers, copolymers of sodium acrylate with sodium maleate, copolymers of sodium acrylate with acrylamide, sodium styrene sulfonate polymers, copolymers of sodium polyisoprene sulfonate with styrene, sodium naphthalene sulfonate polymers, carboxymethyl starch, phosphate-modified starch, carboxymethyl cellulose, sodium alginate, gum arabic, carrageenan, sodium chondroitin sulfate, and sodium hyaluronate.

Examples of the cationic water-soluble polymer compound include a polymer of dimethyldiallylammonium chloride, a polymer of vinyl imidazoline, a polymer of methylvinylimidazolium chloride, a polymer of ethyl acrylate trimethylammonium chloride, a polymer of ethyl methacrylate trimethylammonium chloride, a polymer of acrylamidopropyltrimethylammonium chloride, a polymer of methacrylamidopropyltrimethylammonium chloride, a polymer of epichlorohydrin/dimethylamine, ethylene imine polymers, a quaternized product of a polymer of ethyleneimine, a polymer of allylamine hydrochloride, polylysine, cationic starch, cationized cellulose, chitosan, and derivatives such as copolymer of these with a monomer having a nonionic group or an anionic group.

Examples of the zwitterionic water-soluble polymer compound include copolymers of ethyl acrylate trimethylammonium chloride, acrylic acid and acrylamide, copolymers of ethyl methacrylate trimethylammonium chloride, acrylic acid and acrylamide, and Hoffmann degradation products of acrylamide polymers.

The fiber treatment agent of the present invention may contain the cationic surfactant. Examples of the cationic surfactant include surfactants containing an ammonium group such as halogenated alkyldimethylammonium, and the compound having the chemical formula R⁴R⁵R⁶R⁷N⁺ X⁻ , wherein R⁴, R⁵, R⁶ and R⁷ are independently selected from groups such as alkyl group, aryl group, alkylalkoxy group, arylalkoxy group and alkoxy group, and X is an anion such as a halogen. The cationic surfactant is not limited to these. The fiber treatment agent of the present invention may include an appropriate amount of the cationic surfactant which does not impair the effect of the present invention, but in a more preferred embodiment, a cationic surfactant is desirably not included.

The fiber treatment agent of the present invention may further contain an antibacterial preservative or antibacterial agent. Examples of the antibacterial preservatives include paraoxybenzoic acid alkyl ester, benzoic acid, sodium benzoate, sorbic acid, potassium sorbate, and phenoxyethanol. Examples of the antibacterial agents include benzoic acid, salicylic acid, phenol, sorbic acid, alkyl para-hydroxybenzoates, p-chloro-m-cresol, hexachlorophene, benzalkonium chloride, chlorohexidine chloride, trichlorocarbanilide, photosensitizers and phenoxyethanol.

### [Preparation Method]

The silicone aqueous dispersion of the aforementioned silicone elastomer may be obtained by emulsifying the alkenyl group-containing organopolysiloxane (A-1) with the organopolysiloxane having hydrosilyl groups (A-2) and (A-3) in water (E) using at least one surfactant selected from (B) anionic surfactants and (C) nonionic surfactants, and then a platinum group metal catalyst is added to conduct the addition reaction. The colloidal silica (D) is preferably blended as a colloidal silica dispersion containing water as a dispersing medium as described above, and the colloidal silica (D) is preferably added to the aforementioned aqueous dispersion after the emulsifying step or after the addition reaction step.

Emulsification may be performed using a conventional emulsifier/disperser. Examples of the emulsifier/disperser include a high speed rotation centrifugal radiation type agitator such as homo-disper, a high speed rotation shear type agitator such as homo-mixer, a high pressure injection type emulsifier/disperser such as pressure homogenizer, a colloidal mill and an ultrasonic emulsifier.

When the aforementioned (A) silicone elastomer contains a silicone oil, a silicone resin, an organosilane, an inorganic powder, an organic powder and/or an antioxidant, these should be mixed with the raw material of component (A) in advance.

The platinum group metal catalyst may be added after the emulsifying step as mentioned above, but may also be dissolved in the raw material mixture of component (A) in advance. When the platinum group metal catalyst is added after the emulsifying step, it may be dissolved in a solvent prior to addition. When the platinum group metal catalyst is poorly dispersible in water, the platinum group metal catalyst may be added in a state in which it was dissolved in the (C) nonionic surfactant. When the platinum group metal catalyst was dissolved in the raw material mixture of component (A) in advance, the mixture may be cooled to a low temperature of, for example, 5°C or less in order to restrain the addition reaction until the emulsifying step is completed. The addition reaction may be performed at a normal temperature, for example, from 20 to 25°C. When the reaction is not completed, the reaction may be performed by heating to below 100°C. The stirring time for the reaction is not particularly limited and is typically 1 to 24 hours.

The silicone elastomer obtained by the addition reaction of the components (A-1) with components (A-2) and (A-3) is dispersed in the aqueous dispersion of a silicone elastomer obtained by the aforementioned method. The aqueous dispersion of a silicone elastomer is aqueous and is easily diluted with water. The fiber treatment agent of the present invention is the aqueous dispersion of a silicone elastomer. The fibers are treated with the aqueous dispersion of a silicone elastomer of the present invention (i.e., fiber treatment agent) and dried to form the elastomer coating. The elastomer coating may be tacky, but may not be a gel. An appropriate drying temperature is selected in the range from 1 to 250°C, and the coating can be formed even at room temperature, typically 25°C. The drying time is preferably several seconds to one week. Drying is most preferably performed at room temperature, as a high temperature treatment may damage the fibers. As stated above, the fiber treatment agent of the present invention can impart good coating physical characteristics to fibers, even when it is dried at room temperature. Drying at a high temperature and for a long period of time is not preferable as the silicone elastomer may deteriorate. When coating formability is evaluated, drying may be performed at a temperature less than the boiling point of water (100°C) in order to prevent the generation of bubbles and obtain a smooth coating.

The rubber hardness, the elongation and the tensile strength of the coating obtained from the fiber treatment agent of the present invention are described below. If the rubber hardness is too low, the elongation is too low, and/or the tensile strength is too low, for example, after treating the fibers with the fiber treatment agent, the coating may easily fall off from the fibers by a washing treatment or rubbing.

The coating (hereinafter, referred to as the elastomer coating or the elastomer sheet) consisting of the dry product of the aqueous dispersion of the silicone elastomer (i.e., the fiber treatment agent) of the present invention may have an Asker C rubber hardness of at least 5 at a thickness of 1 mm. The coating may have an elongation at break determined in accordance with JIS (the Japanese Industrial Standards) K 6251 of at least 20% at a thickness of 1 mm, and may have a tensile strength at break determined in accordance with JIS K 6251 of at least 0.05 MPa. This is described in more detail below.

The preparation method of the aforementioned coating (elastomer sheet) is not particularly limited. For example, the fiber treatment agent is poured into a polypropylene tray at an amount to make the thickness after drying about 1 mm and dried at 25°C for 48 hours to obtain the elastomer sheet.

The rubber hardness of the coating (elastomer sheet) may be determined using a Type A Durometer tester according to the method of JIS K 6249. When the Durometer Type A scale rubber hardness is less than 10, the rubber hardness determined by an Asker C tester according to the Society of Rubber Industry, Japan Standards (SRIS-0101) may be at least 5, preferably 10 or more. The upper limit is not particularly limited, but the rubber hardness determined by the Type A Durometer tester may be 60 or smaller, or the rubber hardness determined by Asker C scale may be 80 or smaller.

The elongation at break of a dumbbell shaped #3 specimen of the elastomer sheet determined in accordance with JIS K 6251 is preferably 20% or more, more preferably 50% or more. The upper limit is not particularly limited, but may be set to no more than 1,000%. The tensile strength at break (according to the test method specified in JIS K 6251) of the elastomer sheet is preferably 0.05 MPa or more, and more preferably 0.10 MPa or more. The upper limit is not particularly limited, but may be 5.0 MPa or smaller.

The fiber treatment agent of the present invention may quickly form the coating having elongation and strength on drying at room-temperature. The fiber treatment agent may be used as, for example, water-repellents, water-proof agents, and parting agents for paper, water-repellent and texture improver for textile products, water-proof agents for concrete, mortar and wood, and binders for coating agents containing inorganic particles such as titanium oxide particles.

### EXAMPLES

The present invention will be explained below in further detail with reference to a series of the Examples and the Comparative Examples, although the present invention is in no way limited by these Examples. In the following description, the kinematic viscosity is a value determined at 25°C by an Ostwald viscometer. The term "part" refers to "part by mass".

Each component used in the Examples and Comparative Examples will be described as follows.

### [Raw Materials of the (A) silicone Elastomer]

(A-1') An alkenyl group-containing organopolysiloxane represented by the following general formula (12), having a kinematic viscosity at 25°C of 4,820 mm²/s, and a vinyl content of 0.0064 mol/100 g (the content of each of octamethylcyclotetrasiloxane, decamethylcyclopentasiloxane, and dodecamethylcyclohexanesiloxane is no more than 100 ppm). 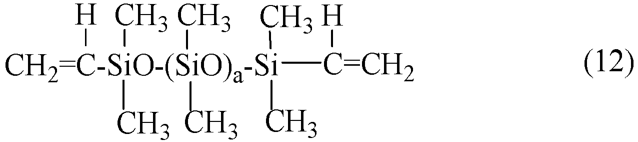 In the formula, a is the number such that the viscosity at 25°C is 4,820 mm²/s.

(A-1") An alkenyl group-containing organopolysiloxane represented by the general formula (12), having a kinematic viscosity at 25°C of 126 mm²/s, and a vinyl content of 0.035 mol/100 g (the content of each of octamethylcyclotetrasiloxane, decamethylcyclopentasiloxane, and dodecamethylcyclohexanesiloxane is no more than 100 ppm). In the aforementioned formula, a is the number such that the viscosity at 25°C is 126 mm²/s.

(A-2') A hydrosilyl group-containing organopolysiloxane represented by the following general formula (13), having a kinematic viscosity at 25°C of 3,450 mm²/s, and a hydrosilyl group content of 0.013 mol/100 g (the content of each of octamethylcyclotetrasiloxane, decamethylcyclopentasiloxane, and dodecamethylcyclohexanesiloxane is no more than 100 ppm). 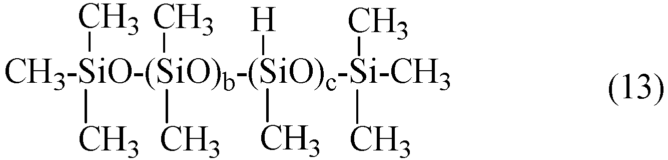 In the formula, b and c are the numbers such that the viscosity at 25°C is 3,450 mm²/s, and the hydrosilyl group content is 0.013 mol/100 g.

(A-2") A hydrosilyl group-containing organopolysiloxane represented by the following general formula (14), having a kinematic viscosity at 25°C of 369 mm²/s, and a hydrosilyl group content of 0.04 mol/100 g (the content of each of octamethylcyclotetrasiloxane, decamethylcyclopentasiloxane, and dodecamethylcyclohexanesiloxane is no more than 100 ppm). 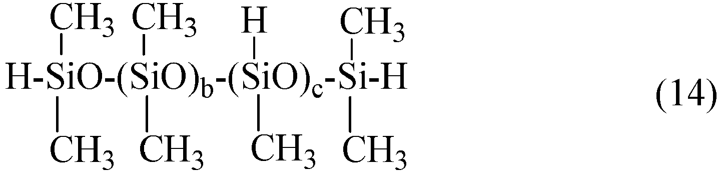 In the formula, b and c are the numbers such that the viscosity at 25°C is 369 mm²/s, and the hydrosilyl group content is 0.04 mol/100 g.

(A-3') A hydrosilyl group-containing organopolysiloxane represented by the following general formula (15), having a kinematic viscosity at 25°C of 3,420 mm²/s, and a hydrosilyl group content of 0.006 mol/100 g (the content of each of octamethylcyclotetrasiloxane, decamethylcyclopentasiloxane, and dodecamethylcyclohexanesiloxane is no more than 100 ppm). 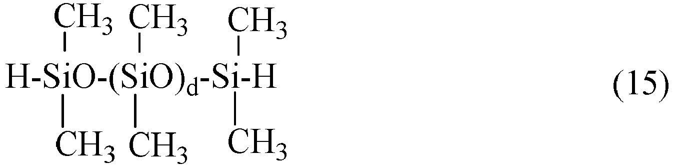 In the formula, d is the number such that the viscosity at 25°C is 3,420 mm²/s.

(A-3") A hydrosilyl group-containing organopolysiloxane represented by the general formula (15), having a kinematic viscosity at 25°C of 47 mm²/s, and a hydrosilyl group content of 0.066 mol/100 g (the content of each of octamethylcyclotetrasiloxane, decamethylcyclopentasiloxane, and dodecamethylcyclohexanesiloxane is no more than 100 ppm). In the formula, d is the number such that the viscosity at 25°C is 47 mm²/s.

### [Surfactant]

### (B) Anionic Surfactant

(B1) Aqueous solution of 30% sodium lauroylmethylalanine (Trade name: NIKKOL Alaninate LN-30, ex Nikko Chemicals Co., Ltd)
(B2) Sodium lauryl phosphate (Trade name: NIKKOL SLP-N, ex Nikko Chemicals Co., Ltd)
(B3) Dodecylbenzene sulfonic acid (Trade name: Neopelex GS-P, ex Kao Corporation)
(B4) Sodium lauryl sulfate (Trade name: NIKKOL SLS, ex Nikko Chemicals Co., Ltd)

### (C) Nonionic surfactant

Polyoxyethylene lauryl ether (Trade name: Emulgen 109P, ex Kao Corporation)

### (D) Colloidal silica

Aqueous dispersion of 20% amount of colloidal silica (silica sol) (Trade name: Snowtex C, ex Nissan Chemical Industries, Ltd., particle size: 10 to 15 nm, pH: 8.5 to 9.0)

### Other Components

(F) A solution of the addition reaction product of maleic anhydride and 3-aminopropyltriethoxysilane in ethanol. The reaction product is a mixture of about 60% of the compounds represented in the following formulas, and the remaining about 40% are oligomers derived therefrom.

(C₂H₅O)₃SiC₃H₆-NHCO-CH = CHCOOH

(C₂H₅O)₃SiC₃H₆NH₃ ^{+ -}OCOCH = CHCOOC₂H₅

### [Preparation Example 1]

### Preparation of Emulsion Composition 1

59.87 parts of (A-1') alkenyl group-containing organopolysiloxane, 26.04 parts of (A-2')hydrosilyl group-containing organopolysiloxane, and 14.09 parts of (A-3') hydrosilyl group-containing organopolysiloxane were uniformly mixed with a homo-mixer, to which 5.0 parts of (C) nonionic surfactant and 6.67 parts of (E) water were then added and emulsified with a homo-mixer. After further stirring with a disper mixer, 87.14 parts by mass of (E) water was added to the mixture, and the mixture was diluted and dispersed by a homo-mixer to obtain an emulsion.

A dissolved mixture of 0.16 parts of a solution of platinum/vinyl-containing siloxane complex in isododecane having a platinum content of 0.5% and 0.16 parts of (C) nonionic surfactant was added to the emulsion thus obtained and stirred at 25°C for two hours to conduct an addition reaction of component (A-1') with components (A-2') and (A-3') to, thereby, obtain an emulsion composition 1 comprising the silicone elastomer thus obtained.

### [Example 1]

### Preparation of Fiber Treatment Agent 1

31.0 parts of (D) aqueous dispersion of 20% amount of colloidal silica, 2.56 parts of component (F) and 1.21 parts of 3-glycidoxypropyltrimethoxysilane were stirred at a stirring speed of 150 rpm for 30 minutes at room temperature, to which 100 parts by mass of the aforementioned emulsion composition 1 was then added. The mixture was further stirred at a stirring speed of 150 rpm for 30 minutes at room temperature to obtain fiber treatment agent 1 which is an aqueous dispersion of a silicone elastomer.

### [Preparation Example 2]

### Preparation of Emulsion Composition 2

55.65 parts of (A-1') alkenyl group-containing organopolysiloxane, 18.15 parts of (A-2') hydrosilyl group-containing organopolysiloxane and 26.20 parts of (A-3') hydrosilyl group-containing organopolysiloxane were uniformly mixed with a homo-mixer, to which 5.0 parts of (C) nonionic surfactant and 6.67 parts of (E) water were then added and emulsified with a homo-mixer. After further stirring with a disper mixer, 87.14 parts by mass of (E) water was added to the mixture, and the mixture was diluted and dispersed by a homo-mixer to obtain an emulsion.

A dissolved mixture of 0.16 parts of a solution of platinum/vinyl-containing siloxane complex in isododecane (platinum content of 0.5%) and 0.16 parts of (C) nonionic surfactant was added to the emulsion thus obtained and stirred at 25°C for two hours to conduct an addition reaction of component (A-1') with components (A-2') and (A-3') to, thereby, obtain emulsion composition 2 comprising the silicone elastomer thus obtained.

### [Example 2]

### Preparation of Fiber Treatment Agent 2

31.0 parts of (D) aqueous dispersion of 20% amount of colloidal silica, 2.56 parts of component (F) and 1.21 parts of 3-glycidoxypropyltrimethoxysilane were stirred at a stirring speed of 150 rpm for 30 minutes at room temperature, to which 100 parts by mass of the aforementioned emulsion composition 2 was then added and further stirred at a stirring speed of 150 rpm for 30 minutes at room temperature to obtain fiber treatment agent 2 which is an aqueous dispersion of a silicone elastomer.

### [Preparation Example 3]

### Preparation of Emulsion Composition 3

51.99 parts of (A-1') alkenyl group-containing organopolysiloxane, 11.31 parts of (A-2') hydrosilyl group-containing organopolysiloxane and 36.70 parts of (A-3') hydrosilyl group-containing organopolysiloxane were uniformly mixed with a homo-mixer, to which 5.0 parts of (C) nonionic surfactant and 6.67 parts of (E) water were then added and emulsified with a homo-mixer. After further stirring with a disper mixer, 87.14 parts by mass of (E) water was added to the mixture, and the mixture was diluted and dispersed by a homo-mixer to obtain an emulsion.

A dissolved mixture of 0.16 parts of a solution of platinum/vinyl-containing siloxane complex in isododecane (platinum content of 0.5%) and 0.16 parts of (C) nonionic surfactant was added to the emulsion thus obtained and stirred at 25°C for two hours to conduct an addition reaction of component (A-1') with components (A-2') and (A-3') to, thereby, obtain emulsion composition 3 comprising the silicone elastomer thus obtained.

### [Example 3]

### Preparation of Fiber Treatment Agent 3

31.0 parts of (D) aqueous dispersion of 20% amount of colloidal silica, 2.56 parts of component (F), and 1.21 parts of 3-glycidoxypropyltrimethoxysilane were stirred at a stirring speed of 150 rpm for 30 minutes at room temperature, to which 100 parts by mass of the aforementioned emulsion composition 3 was then added and further stirred at a stirring speed of 150 rpm for 30 minutes at room temperature to obtain fiber treatment agent 3 which is an aqueous dispersion of a silicone elastomer.

### [Preparation Example 4]

### Preparation of Emulsion Composition 4

48.78 parts of (A-1') alkenyl group-containing organopolysiloxane, 5.30 parts of (A-2') hydrosilyl group-containing organopolysiloxane, and 45.92 parts of (A-3') hydrosilyl group-containing organopolysiloxane were uniformly mixed with a homo-mixer, to which 5.0 parts of (C) nonionic surfactant and 6.67 parts of (E) water were then added and emulsified with a homo-mixer. After further stirring with a disper mixer, 87.14 parts by mass of (E) water was added to the mixture, and the mixture was diluted and dispersed by a homo-mixer to obtain an emulsion.

A dissolved mixture of 0.16 parts of a solution of platinum/vinyl-containing siloxane complex in isododecane (platinum content of 0.5%) and 0.16 parts of (C) nonionic surfactant was added to the emulsion thus obtained and stirred at 25°C for two hours to conduct an addition reaction of component (A-1') with components (A-2') and (A-3') to, thereby, obtain emulsion composition 4 comprising the silicone elastomer thus obtained.

### [Example 4]

### Preparation of fiber treatment agent 4

31.0 parts of (D) aqueous dispersion of 20% amount of colloidal silica, 2.56 parts of component (F), 1.21 parts of 3-glycidoxypropyltrimethoxysilane were stirred at a stirring speed of 150 rpm for 30 minutes at room temperature, to which 100 parts by mass of the aforementioned emulsion composition 4 was then added and further stirred at a stirring speed of 150 rpm for 30 minutes at room temperature to obtain fiber treatment agent 4 which is an aqueous dispersion of a silicone elastomer.

### [Preparation Example 5]

### Preparation of emulsion composition 5

57.76 parts of (A-1") alkenyl group-containing organopolysiloxane, 17.44 parts of (A-2") hydrosilyl group-containing organopolysiloxane and 24.80 parts of (A-3") hydrosilyl group-containing organopolysiloxane were uniformly mixed with a homo-mixer, to which 3.33 parts of (B1) anionic surfactant, 23.64 parts of (E) water and 1.82 parts of phenoxyethanol were then added and emulsified with a homo-mixer. 52.73 parts by mass of (E) water was added to the mixture, diluted and dispersed by a homo-mixer, and subjected to high-pressure treatment once at a treatment pressure of 100 MPa by a high-pressure emulsifier to obtain an emulsion.

A dissolved mixture of 0.16 parts of a solution of platinum/vinyl-containing siloxane complex in isododecane (platinum content of 0.5%) and 0.16 parts of (C) nonionic surfactant was added to the emulsion thus obtained and stirred at 25°C for 12 hours to conduct an addition reaction of the aforementioned component (A-1") with the aforementioned components (A-2") and (A-3") to, thereby, obtain emulsion composition 5 comprising the silicone elastomer thus obtained.

### [Example 5]

### Preparation of fiber treatment agent 5

100 parts of the aforementioned emulsion composition 5 and 68.75 parts of (D) aqueous dispersion of 20% amount of colloidal silica were stirred at a stirring speed of 150 rpm for 30 minutes at room temperature to obtain fiber treatment agent 5 which is an aqueous dispersion of a silicone elastomer.

### [Example 6]

### Preparation of fiber treatment agent 6

100 parts of the aforementioned emulsion composition 5 and 17.19 parts of (D) aqueous dispersion of 20% amount of colloidal silica were stirred at a stirring speed of 150 rpm for 30 minutes at room temperature to obtain fiber treatment agent 6 which is an aqueous dispersion of a silicone elastomer.

### [Preparation Example 6]

### Preparation of emulsion composition 6

57.76 parts of (A-1") alkenyl group-containing organopolysiloxane, 17.44 parts of (A-2") hydrosilyl group-containing organopolysiloxane, and 24.80 parts of (A-3") hydrosilyl group-containing organopolysiloxane were uniformly mixed with a homo-mixer, to which 1.0 parts of (B2) anionic surfactant, 25.96 parts of (E) water, and 1.82 parts of phenoxyethanol were then added and emulsified with a homo-mixer. 52.73 parts by mass of (E) water was added to the mixture, diluted and dispersed by a homo-mixer, and subjected to high-pressure treatment once at a treatment pressure of 100 MPa by a high-pressure emulsifier to obtain an emulsion.

A dissolved mixture of 0.16 parts of a solution of platinum/vinyl-containing siloxane complex in isododecane (platinum content of 0.5%) and 0.16 parts of (C) nonionic surfactant was added to the emulsion thus obtained and stirred at 25°C for 12 hours to conduct an addition reaction of the aforementioned component (A-1") with the aforementioned components (A-2") and (A-3") to, thereby, obtain emulsion composition 6 comprising the silicone elastomer thus obtained.

### [Example 7]

### Preparation of fiber treatment agent 7

100 parts of the aforementioned emulsion composition 6 and 68.75 parts of (D) aqueous dispersion of colloidal silica were stirred at a stirring speed of 150 rpm for 30 minutes at room temperature to obtain fiber treatment agent 7 which is an aqueous dispersion of a silicone elastomer.

### [Example 8]

### Preparation of fiber treatment agent 8

100 parts of the aforementioned emulsion composition 6 and 17.19 parts of (D) aqueous dispersion of colloidal silica were stirred at a stirring speed of 150 rpm for 30 minutes at room temperature to obtain fiber treatment agent 8 which is an aqueous dispersion of a silicone elastomer.

### [Comparative Preparation Example 1]

### Preparation of emulsion composition 7

59.87 parts of (A-1') alkenyl group-containing organopolysiloxane, 26.04 parts of (A-2') hydrosilyl group-containing organopolysiloxane and 14.09 parts of (A-3') hydrosilyl group-containing organopolysiloxane were uniformly mixed with a homo-mixer, to which 8.5 parts of (C) nonionic surfactant, and 6.67 parts of (E) water were then added and emulsified with a homo-mixer. After further stirring with a disper mixer, 87.14 parts by mass of (E) water was added to the mixture, and the mixture was diluted and dispersed by a homo-mixer to obtain an emulsion.

A dissolved mixture of 0.16 parts of a solution of platinum/vinyl-containing siloxane complex in isododecane (platinum content of 0.5%) and 0.16 parts of (C) nonionic surfactant was added to the emulsion thus obtained and stirred at 25°C for two hours to conduct an addition reaction of component (A-1') with components (A-2') and (A-3') to, thereby, obtain emulsion composition 7 comprising the silicone elastomer thus obtained.

### [Comparative Example 1]

### Preparation of fiber treatment agent 9

31.0 parts of (D) aqueous dispersion of colloidal silica, 2.56 parts of component (F) and 1.24 parts of 3-glycidoxypropyltrimethoxysilane were stirred at a stirring speed of 150 rpm for 30 minutes at room temperature, to which 100 parts of the aforementioned emulsion composition 7 was then added and further stirred at a stirring speed of 150 rpm for 30 minutes at room temperature to obtain fiber treatment agent 9 which is an aqueous dispersion of a silicone elastomer.

### [Comparative Preparation Example 2]

### Preparation of emulsion composition 8

45.94 parts of (A-1') alkenyl group-containing organopolysiloxane and 54.06 parts of (A-3') hydrosilyl group-containing organopolysiloxane were uniformly mixed with a homo-mixer, to which 5.0 parts of (C) nonionic surfactant and 6.67 parts of (E) water were then added and emulsified with a homo-mixer. After further stirring with a disper mixer, 87.14 parts by mass of (E) water was added to the mixture, and the mixture was diluted and dispersed by a homo-mixer to obtain an emulsion.

A dissolved mixture of 0.16 parts of a solution of platinum/vinyl-containing siloxane complex in isododecane (platinum content of 0.5%) and 0.16 parts of (C) nonionic surfactant was added to the emulsion thus obtained and stirred at 25°C for two hours to conduct an addition reaction of the aforementioned component (A-1') and the aforementioned component (A-3') to, thereby, obtain emulsion composition 8 comprising the silicone elastomer thus obtained.

### [Comparative Example 2]

### Preparation of fiber treatment agent 10

31.0 parts of (D) aqueous dispersion of colloidal silica, 2.56 parts of component (F) and 1.24 parts of 3-glycidoxypropyltrimethoxysilane were stirred at a stirring speed of 150 rpm for 30 minutes at room temperature, to which 100 parts of the aforementioned emulsion composition 8 was then added and further stirred at a stirring speed of 150 rpm for 30 minutes at room temperature, to obtain fiber treatment agent 10 which is an aqueous dispersion of a silicone elastomer.

### [Comparative Example 3]

### Preparation of fiber treatment agent 11

31.0 parts of (E) water, 2.56 parts of component (F) and 1.24 parts of 3-glycidoxypropyltrimethoxysilane were stirred at a stirring speed of 150 rpm for 30 minutes at room temperature, to which 100 parts of the aforementioned emulsion composition 1 was then added and further stirred at a stirring speed of 150 rpm for 30 minutes at room temperature, to obtain fiber treatment agent 11 which is an aqueous dispersion of a silicone elastomer.

### [Comparative Preparation Example 3]

### Preparation of emulsion composition 9

An aqueous solution obtained by diluting 1.0 part of (B3) anionic surfactant with 8.97 parts of (C) water and an aqueous solution obtained by diluting 1.0 part of (B4) anionic surfactant with 8.97 parts of (C) water were added to a mixture of 99.64 parts of octamethylcyclotetrasiloxane and 0.36 parts of phenyltriethoxysilane and emulsified with a homo-mixer. 79.35 parts of (C) water was added to the mixture, diluted and dispersed by a homo-mixer, and subjected to high-pressure treatment once at a treatment pressure of 100 MPa by a high-pressure emulsifier to obtain an emulsion. The emulsion thus obtained was stirred at 50°C for 2 hours, and then stirred at 15°C for 12 hours, neutralized by the addition of 2.07 parts of 10% sodium carbonate aqueous solution, and 1.0 part of (B4) anionic surfactant was added and dissolved by stirring to obtain emulsion composition 9.

### [Comparative Example 4]

### Preparation of fiber treatment agent 12

31.0 parts of (D) aqueous dispersion of colloidal silica, 2.56 parts of component (F) and 1.24 parts of 3-glycidoxypropyltrimethoxysilane were stirred at a stirring speed of 150 rpm for 30 minutes at room temperature, and then 100 parts of the aforementioned emulsion composition 9 was added and further stirred at a stirring speed of 150 rpm for 30 minutes at room temperature to, thereby, obtain fiber treatment agent 12 which is an aqueous dispersion of a silicone elastomer.

### [Comparative Preparation Example 4]

### Preparation of emulsion composition 10

50.98 parts of (A-1') alkenyl group-containing organopolysiloxane and 49.02 parts of (A-2') hydrosilyl group-containing organopolysiloxane were uniformly mixed with a homo-mixer, and then 3.33 parts of (B') anionic surfactant, 23.64 parts of (E) water, and 1.82 parts of phenoxyethanol were added and emulsified with a homo-mixer. 52.73 parts by mass of (E) water was added to the mixture, diluted and dispersed by a homo-mixer, and subjected to high-pressure treatment once at a treatment pressure of 100 MPa by a high-pressure emulsifier to obtain an emulsion.

A dissolved mixture of 0.16 parts of a solution of platinum/vinyl-containing siloxane complex in isododecane (platinum content of 0.5%) and 0.16 part of (C) nonionic surfactant was added to the emulsion thus obtained and stirred at 25°C for 12 hours to conduct an addition reaction of the aforementioned component (A-1") and the aforementioned component (A-2") to, thereby, obtain emulsion composition 10 comprising the silicone elastomer thus obtained.

### [Comparative Example 5]

### Preparation of fiber treatment agent 13

100 parts of the aforementioned emulsion composition 10 and 17.19 parts of (D) aqueous dispersion of colloidal silica were stirred at a stirring speed of 150 rpm for 30 minutes at room temperature to obtain fiber treatment agent 13 which is an aqueous dispersion of a silicone elastomer.

The physical properties and characteristics of each of the aforementioned fiber treatment agents were determined or evaluated in accordance with the methods shown below. The results are shown in Table 1.

### [Physical properties of the coating]

20 g of each fiber treatment agent was poured into a polypropylene resin disposable tray (150 mm × 105 mm × 10 mm) and dried at 25°C for 48 hours to prepare a coating having a thickness of about 1 mm. The hardness, the tensile strength (MPa•s) and the elongation at break (%) of the coating thus obtained were determined according to JIS K6249. The hardness is the Durometer Type A scale rubber hardness. In Comparative Example 3, the rubber hardness determined by Durometer Type A tester was less than 10 and, therefore, the rubber hardness was determined by an Asker C tester according to the Society of Rubber Industry, Japan Standards (SRIS).

### [Coating formability]

The state of the coating obtained as stated above was evaluated based on the following criteria.
A: a uniform coating having a strength which could be easily peeled off from the disposable tray was formed.
B: a uniform coating was formed, but could not be peeled off from the disposable tray.
C: a uniform coating was not formed.

### [Washing durability]

Ion-exchange water was added to each fiber treatment agent and diluted to a solid content of 2% to prepare a test solution. After immersing a polyester / cotton broad cloth (65% of polyester / 35% of cotton, ex Tanigashira Shoten) in the test solution for 10 seconds, a roller was used to squeeze the cloth to a squeeze percentage of 100%, and then the cloth was dried at 130°C for 2 minutes.

The treatment cloth thus obtained was washed once with a washing machine by a method according to JIS LO217 103. The amount of silicone adhered to the fiber surface of the treatment cloth when not washed, and when washed once, was determined with a fluorescent X-ray analyzer (ex Rigaku Corporation), and the silicone residual percentage (%) was calculated by comparing aforementioned two conditions.

### [Amount of the cyclic siloxane in emulsion]

After extracting (with shaking for 3 hours) 0.1 g of each fiber treatment agent with 10 ml of acetone containing 20 ppm (mass) of tetradecane as an internal standard, and after being left standing overnight, the acetone layer was sampled and gas chromatography analysis was performed to quantify the octamethylcyclotetrasiloxane, decamethylcyclopentasiloxane and dodecamethylcyclohexanesiloxane.

**Table 1**

| Composition, part by mass | | | Ex. 1 | Ex. 2 | Ex. 3 | Ex. 4 | Ex. 5 | Ex. 6 | Ex. 7 | Ex. 8 | Com. Ex. 1 | Com. Ex. 2 | Com. Ex. 3 | Com. Ex. 4 | Com. Ex. 5 |
|---|---|---|---|---|---|---|---|---|---|---|---|---|---|---|---|
| (A) Silicone Elastomer raw material | | (A-1') | 59.87 | 55.65 | 51.99 | 48.78 | | | | | 59.87 | 45.94 | 59.87 | | |
| | | (A-1") | | | | | 57.76 | 57.76 | 57.76 | 57.76 | | | | | 50.98 |
| | | (A-2') | 26.04 | 18.15 | 11.31 | 5.30 | | | | | 26.04 | | 26.04 | | |
| | | (A-2") | | | | | 17.44 | 17.44 | 17.44 | 17.44 | | | | | 49.02 |
| | | (A-3') | 14.09 | 26.20 | 36.70 | 45.92 | | | | | 14.09 | 54.06 | 14.09 | | |
| | | (A-3") | | | | | 24.80 | 24.80 | 24.80 | 24.80 | | | | | |
| Ratio of number SiH of A-2 and A-3/ number SiVi of A-1 | | | 1.10 | 1.10 | 1.10 | 1.10 | 1.15 | 1.15 | 1.15 | 1.15 | 1.10 | 1.10 | 1.10 | - | 1.10 |
| (B) Anionic Surfactant | | (B1) | | | | | 1.0 | 1.0 | | | | | | | 1.0 |
| | | (B2) | | | | | | | 1.0 | 1.0 | | | | | |
| | | (B3) | | | | | | | | | | | | 1.00 | |
| | | (B4) | | | | | | | | | | | | 2.00 | |
| (C) Nonionic Surfactant | | | 5.16 | 5.16 | 5.16 | 5.16 | 0.16 | 0.16 | 0.16 | 0.16 | 8.66 | 5.16 | 5.16 | 0 | 0.16 |
| (D) Colloidal Silica | | | 12.34 | 12.34 | 12.34 | 12.34 | 24.75 | 6.19 | 24.75 | 6.19 | 12.56 | 12.34 | 0 | 12.53 | 6.19 |
| (E) Water | | | 143.18 | 143.18 | 143.18 | 143.18 | 103.44 | 177.68 | 103.44 | 177.68 | 144.05 | 143.18 | 155.53 | 149.29 | 177.68 |
| Octamethylcyclotetrasiloxane | | | | | | | | | | | | | | 99.64 | |
| Phenyltriethoxvsilane | | | | | | | | | | | | | | 0.36 | |
| Coating physical characteristics | Hardness | | 25 | 28 | 21 | 20 | 71 | 11 | 58 | 19 | - | - | 2 | 15 | 17 |
| | Tensile strength MPa | | 0.34 | 0.45 | 0.44 | 0.33 | 1.67 | 0.61 | 2.45 | 0.69 | - | - | 0.10 | 0.40 | 0.50 |
| | Elongation % | | 45 | 171 | 48 | 75 | 265 | 603 | 436 | 430 | - | - | 43 | 680 | 15 |
| Coating formability | | | A | A | A | A | A | A | A | A | c | c | A | A | A |
| Silicone residual ratio % | | | 36 | 33 | 39 | 48 | 62 | 75 | 62 | 69 | 28 | 27 | 30 | 45 | 12 |
| D₄ content ppm/emulsion | | | < 100 | < 100 | < 100 | < 100 | < 100 | < 100 | < 100 | < 100 | < 100 | < 100 | < 100 | 15,840 | < 100 |
| D₅ content ppm/emulsion | | | < 100 | < 100 | < 100 | < 100 | < 100 | < 100 | < 100 | < 100 | < 100 | < 100 | < 100 | 12,410 | < 100 |
| D₆ content ppm/emulsion | | | < 100 | < 100 | < 100 | < 100 | < 100 | < 100 | < 100 | < 100 | < 100 | < 100 | < 100 | 4,680 | < 100 |

The compositions described in Table 1 show the value (parts by mass) relative to a total 100 parts by mass of component (A). Comparative Example 4 describes the values for a total of 100 parts by mass of octamethylcyclotetrasiloxane and phenyltriethoxysilane. A 30% aqueous solution was used for component (B1) and a 20% dispersion was used for component (D), but the amounts of components (B1) and (D) in Table 1 are the respective active ingredient amounts. In Table 1, D₄ is octamethylcyclotetrasiloxane, D₅ is decamethylcyclopentasiloxane, and D₆ is dodecamethylcyclohexanesiloxane.

As shown in Table 1, the composition of Comparative Example 1 comprised the (C) nonionic surfactant in a large amount, so that tack derived from the component was observed, and cracks were numerous in the coating obtained. Comparative Example 2 had a composition free of component (A-2) and did not form a coating. Comparative Example 3 had a composition free of (D) colloidal silica and the coating obtained could be peeled off from the disposable tray, but had the low hardness. Comparative Example 4 provided a uniform coating which could easily be peeled off from the disposable tray, but contained a large amount of cyclic siloxanes in the fiber treatment agent. Comparative Example 5 had a composition free of (A-3) and provided a coating having sufficient hardness, but was not extensible.

In contrast, the fiber treatment agent of Examples 1 to 8 have a content of cyclic siloxanes of less than 100 ppm. A uniform coating having a strength which can easily be peeled off from a disposable tray was formed by drying the fiber treatment agent at room temperature.

### INDUSTRIAL APPLICABILITY

The fiber treatment agent of the present invention may easily form a coating by drying at room temperature. Therefore, it is not necessary to contain an organotin compound. The coating thus obtained has excellent hardness, tensile strength, and elongation, so that the fiber treatment agent is suitable as a fiber treatment agent for coating on the surface of various substrates or a coating agent.

## Claims

1. A fiber treatment agent comprising
100 parts by mass of (A) a silicone elastomer which is an addition-reaction product of the following component (A-1) with the following components (A-2) and (A-3):
(A-1) an alkenyl group-containing organopolysiloxane having two or more alkenyl groups per molecule,
(A-2) an organohydrogen polysiloxane having three or more hydrosilyl groups per molecule,
(A-3) a linear diorganohydrogen polysiloxane having hydrosilyl groups only at both molecular chain terminals;
0.1 to 8 parts by mass of at least one surfactant selected from (B) anionic surfactants and (C) nonionic surfactants,
3 to 35 parts by mass of (D) colloidal silica, and
15 to 200 parts by mass of (E) water, relative to total 100 parts by mass of components (A) and (D).

2. The fiber treatment agent according to claim 1, wherein the mass ratio of component (A-2) to component (A-3), (A-2):(A-3), is 5:95 to 90:10 in component (A).

3. The fiber treatment agent according to claim 1 or claim 2, wherein the ratio of the total number of the hydrosilyl groups in components (A-2) and (A-3) to the number of the alkenyl groups in component (A-1) is 0.5 to 2.0.

4. The fiber treatment agent according to any one of claims 1 to 3, wherein the amount of (B) anionic surfactant is 0 to 5 parts by mass, the amount of (C) nonionic surfactant is 0 to 8 parts by mass, and the total amount of components (B) and (C) is 0.1 to 8 parts by mass.

5. A coating which is a dry product of the fiber treatment agent according to any one of claims 1 to 4.

6. The coating according to claim 5, having an Asker C rubber hardness of at least 5 determined at a thickness of 1 mm in accordance with the Society of Rubber Industry, Japan Standards (SRIS) .

7. The coating according to claim 5 or claim 6, having an elongation at break of at least 20% determined at a thickness of 1 mm in accordance with JIS K 6249, and a tensile strength of at least 0.05 MPa determined at a thickness of 1 mm in accordance with JIS K 6251.
